# EUROPEAN PATENT SPECIFICATION

(11) **EP 2 094 198 B1**
(45) Date of publication and mention of the grant of the patent: **12.06.2013**
(21) Application number: 07844019.5
(22) Date of filing: 09.10.2007
(51) Int. Cl.: A61F 2/38

(54) **MOBILE/FIXED PROSTHETIC KNEE SYSTEMS**
MOBILE/FIXIERTE KNIEPROTHESENSYSTEME
SYSTÈMES DE GENOUX PROTHÉTIQUES MOBILES/FIXES

(30) Priority: 13.10.2006 US 829430 P; 13.10.2006 US 829432 P; 21.09.2007 US 859442
(43) Date of publication of application: 02.09.2009
(73) Proprietor: DePuy Products, Inc., Warsaw, IN 46581 (US)
(72) Inventor: AUGER, Daniel D., Fort Wayne, IN 46814 (US); HAZEBROUCK, Stephen A., Winona Lake, IN 46590 (US); BARNETT, Gary D., Wabash, IN 46992 (US); KOMISTEK, Richard, Knoxville, TN 37922 (US); DENNIS, Douglas A., Littleton, CO 80128 (US); DUTIEL, Scott E., Fort Wayne, IN 46814 (US); OUTTEN, Joel T., Honolulu, HI 96815 (US); TO, Gary, Knoxville, TN 37996 (US); ZANNIS, Anthony D., Fort Wayne, IN 46845 (US)
(74) Representative: Belcher, Simon James
(86) International application number: PCT/US2007/080792
(87) International publication number: WO 2008/048819

(56) References cited:
- US-A- 4 501 031
- US-A1- 2003 009 232
- US-A1- 2004 034 432
- US-A1- 2005 246 027

## Description

### TECHNICAL FIELD

The present invention relates generally to a knee prosthesis. Specifically, the present invention relates to the tibial and bearing components of a knee prosthesis. The closest prior art is US-A-2004/034432 which defines the preamble of claim 1.

### BACKGROUND

Movement (e.g., flexion and extension) of the natural human knee involves movements of the femur and the tibia. Specifically, during flexion and extension, the distal end of the femur and the proximal end of the tibia articulate relative to one another through a series of complex movements. Damage (e.g., trauma) or disease can deteriorate the bones, articular cartilage, and ligaments of the knee, which can ultimately affect the ability of the natural knee to function in such a manner. As a result, knee prostheses have been developed and implanted into surgically prepared ends of the femur and tibia.

A typical knee prosthesis for a total knee replacement, for example, includes a tibial component or tibial tray coupled to the patient's tibia, a femoral component coupled to the patient's femur, and a bearing component (or tibial insert) positioned between the tibial tray and the femoral component and including a bearing surface to accommodate the condyles of the femoral component. In some situations, it may be desirable that the tibial insert rotate relative to the tibial tray. Such rotation more closely replicates the motion of the patient's natural anatomy. In other cases, however, it may be desirable to prevent the tibial insert from rotating relative to the tibial tray. For example, various ligaments which support the knee may be compromised or damaged. In such a case, rotation of the tibial insert relative to the tibial tray may create an unstable knee. As such, a surgeon will decide on a case-by- case basis whether to use a rotating or non-rotating tibial assembly. This decision may be made pre-operatively or intra-operatively, for example. Additionally, it may be desirable to change a rotating tibial insert to a non-rotating tibial insert during a revision type surgery, for example.

### SUMMARY

The invention provides an orthopaedic prosthesis assembly as defined in claim 1.

In some embodiments, the first slot may define a closed path.

In some embodiments, the tibial tray may include a second slot defined in the side surface. In such embodiments, the first and second slots may be defined in the anterior side of the side surface. Additionally, the tibial insert may include a second tab extending inwardly from the rim and being received in the second slot.

In some embodiments, the tibial tray may also include a second slot and a third slot defined in the side surface. In such embodiments, the first slot maybe defined on the medial side of the tibial tray, the second slot may be defined on the lateral side of the tibial tray, and the third slot may be defined on the anterior side of the tibial tray. Additionally, the tibial insert may include a second tab and a third tab extending inwardly from the rim. The second tab may be received in the second slot and the third tab may be received in the third slot.

Further, in some embodiments, the tibial tray may include a cavity defined in the platform. The cavity may be being defined by, for example, a sidewall. The tibial insert may include a stem extending downwardly from the bottom surface. The stem may be received in the cavity of the tibial tray. Additionally, in some embodiments, the orthopaedic prosthesis assembly may include a metal ring secured to the stem. In such embodiments, the sidewall of the cavity and the metal ring may corresponding tapers such that the sidewall of the cavity and the metal ring are in contact and form a friction lock therebetween. For example, the taper of the sidewall and the taper of the metal ring may be embodied as Morse tapers.

In some embodiments, the orthopaedic prosthesis assembly may include a rotating tibial insert. In such embodiments, the tibial insert may be removably coupled to the tibial tray and the rotating tibial insert may be configured to be coupled to the tibial tray when the tibial insert is removed therefrom. The rotating tibial insert may be free to rotate about an axis relative to the tibial tray when coupled thereto. Additionally, in such embodiments wherein the tibial tray includes a cavity defined in the platform, the rotating tibial insert may include a stem extending from a bottom surface and configured to be received in the cavity of the tibial tray when the rotating tibial insert is coupled thereto.

Additionally, in some embodiments, the tibial tray may include a post extending upwardly from the upper surface. The tibial insert may include an aperture defined in the bottom surface of the tibial insert. The post of the tibial tray may be received in the aperture when the tibial insert is coupled to the tibial tray. In such embodiments, the rotating tibial insert may include an aperture defined in a bottom surface. The post of the tibial tray may be received in the aperture when the rotating tibial insert is coupled to the tibial tray.

### BRIEF DESCRIPTION OF THE DRAWINGS

The detailed description refers to the following figures, in which:
FIG. 1 is an exploded perspective view of another embodiment of an orthopaedic prosthesis assembly including a non-rotating tibial insert;
FIG. 2 is a perspective view of the orthopaedic prosthesis assembly of FIG. 1 shown in an assembled configuration;
FIG. 3 is an exploded perspective view of the orthopaedic prosthesis assembly of FIG. 1 including a rotating tibial insert;
FIG. 4 is a perspective view of the orthopaedic prosthesis assembly of FIG. 3 shown in an assembled configuration;

### DETAILED DESCRIPTION OF THE DRAWINGS

Various prosthetic knee systems are described within the present disclosure. Such prosthetic knee systems may include one or more tibial trays, one or more tibial inserts, and/or one or more locking mechanisms or other components associated with the aforementioned tray(s) and insert(s). A first combination of these components of the prosthetic knee systems disclosed herein provides a rotating tibial assembly whereby the tibial insert is able to rotate about a longitudinal axis relative to the tibial tray. A second combination of the components of the prosthetic knee systems disclosed herein provides a non-rotating or fixed knee assembly whereby the tibial insert is fixed relative to the tibial tray and is not able to rotate about the longitudinal axis. As such, many of the knee prosthetic systems disclosed herein include components which may be arranged to provide for both a rotating knee assembly and a non-rotating knee assembly.

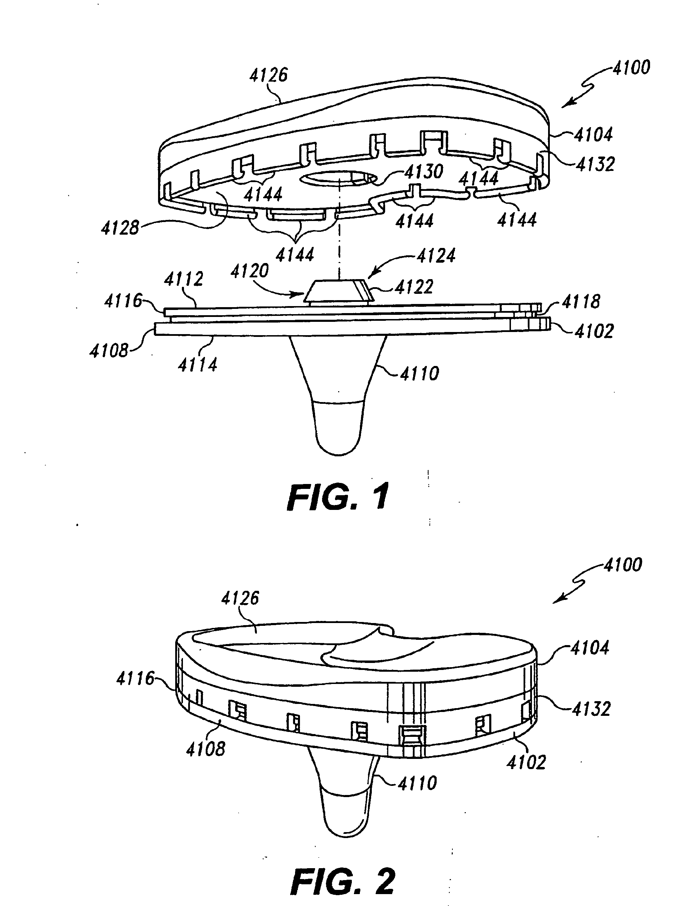

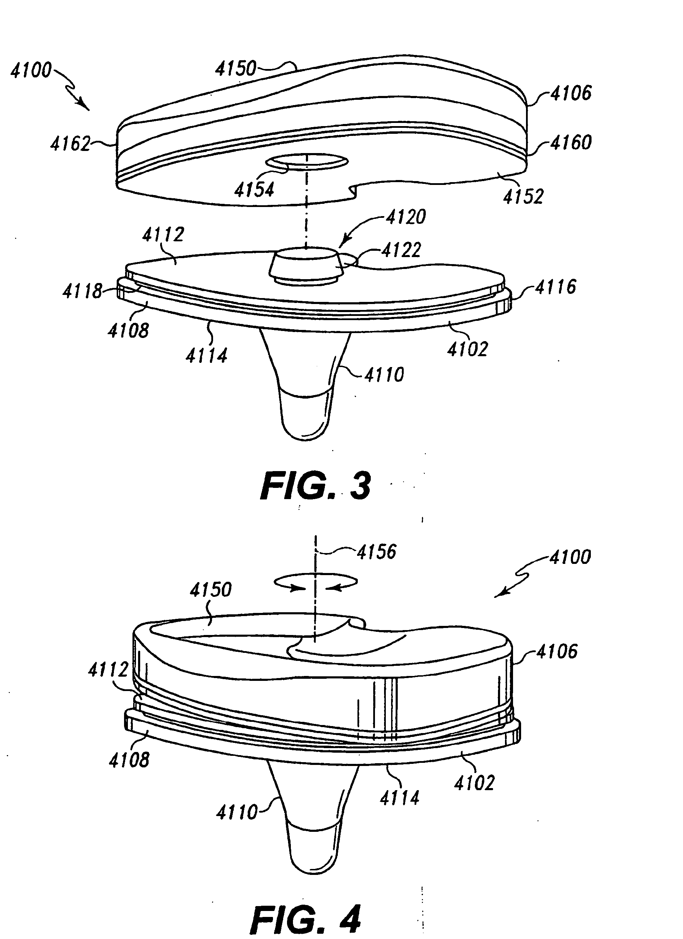

Referring to the drawings, FIGS. 1 to 4 show a prosthetic knee system 4100 which includes a tibial tray 4102, a fixed or non-rotating tibial insert 4104 (see FIGS. 1 and 2) and a rotating tibial insert 4106 (see FIGS. 3 and 4). The tibial inserts 4104, 4106 are illustratively formed from a polymer material, but may be formed from other materials, such as a ceramic material, a metallic material, a bio- engineered material, or the like, in other embodiments. Similarly, the tibial tray 4102 is illustratively formed from a metallic material, but may be formed from other materials, such as a ceramic material, a polymer material, a bio-engineered material, or the like, in other embodiments.

The tibial tray 4102 includes a platform 4108 and a stem 4110. The platform 4108 includes an upper surface 4112, a bottom surface 4114, and a side surface 4116 extending between the upper surface 4112 and the bottom surface 4114. The stem 4110 extends downwardly from the bottom surface 4114 of the platform 4108. The platform 4108 includes a slot 4118 defined in the side surface 4116. Illustratively, the slot 4118 is defined along the length of the side surface 4116 and defines a closed path. However, in other embodiments, the slot 4118 may be embodied as a slot defining an open path, be defined only on particular sections of the side surface 4116, and/or be embodied as a number of smaller slots. The platform 4108 also includes a post 4120 extending upwardly from the upper surface 4112. The post 4120 includes a flange 4122 defined at a proximal end 4124. Illustratively, the flange 4122 includes a upwardly narrowing taper, but flanges having other configurations may be used in other embodiments.

In use, the tibial tray 4102 is configured to be coupled to a surgically- prepared surface of the proximal end of a patient's tibia (not shown). When the tibial tray 4102 is so coupled, the stem 4110 is embedded in patient's tibia to thereby secure the tibial tray 4102 to the patient's bone. In some embodiments, a stem extension (not shown) may include coupled to the stem 4110 to increase the overall length of the stem 4110 and improve the stability of the tibial tray relative to the patient's bony anatomy.

The tibial insert 4104 includes an upper bearing surface 4126 and a bottom surface 4128. The upper bearing surface 4126 is configured to contact a pair of natural or prosthetic femoral condyles of the patient. The bottom surface 4128 includes an aperture 4130 defined therein. As discussed below, the aperture 4130 is configured to receive the post 4120 defined on the upper surface 4112 of the platform 4108 of the tibial tray 4102. The tibial insert 4104 also includes a skirt or rim 4132 extending downwardly from the bottom surface 4128. The rim 4132 includes a number of tabs 4144 extending inwardly. Illustratively, the rim 4132 includes a number of individual downwardly extending sections. Each section includes a separate inwardly extending tab 4132.

As illustrated in FIG. 2, the tibial insert 4104 is configured to be coupled to the tibial tray 4102 in use. To do so, the tibial insert 4104 is positioned on the upper surface 4112 of the platform 4108 such that the post 4120 is received in the aperture 4130 defined in the bottom surface 4116 of the tibial insert 4104. Additionally, the tabs 4144 are received in the slot 4118 defined in the side surface 4116 of the platform 4108 of the tibial tray 4102. When so coupled, the bottom surface 4128 of the tibial insert 4104 is in contact with the upper surface 4112 of the platform 4108 of the tibial tray 4102. In addition, when the non-rotating tibial insert 4104 is coupled to the tibia tray 4102 as shown in FIG. 2, the rim 4132 surrounds the side surface 4116 of the platform 4108 of the tibial tray 4102. The slot 4118 of the tibial tray 4102 and the rim 4132 and tabs 4144 of the rotating tibial insert 4104 cooperate to restrict or prevent rotation of the tibial insert 4104 relative to the tibial tray 4102, to reduce micro-motion between the tibial insert 4104 and the tibial tray 4102, and/or to prevent lift-off of the tibial insert 4104 relative to the tibial tray 4102.

As shown in FIG. 3 and 4, the rotating tibial insert 4104 may be used with the tibial tray 4102 in place of the non-rotating tibial insert 4104. In some embodiments, the rotating tibial insert 4106 is separate from the rotating tibial insert 4104 and includes an upper bearing surface 4150, a bottom surface 4152, an aperture 4154 defined in the bottom surface 4150 similar to the upper bearing surface 4126, the bottom surface 4128, and the aperture 4130 of the non-rotating tibial insert 4104. However, in other embodiments, the rim 4132 of the non-rotating tibial insert 4104 is configured to be removed therefrom to selectively change the non-rotating tibial insert 4104 into a rotating tibial insert. It should be appreciated that, in such embodiments, the non-rotating tibial insert 4104 and the rotating tibial insert 4106 are the same tibial insert. Additionally, in such embodiments, the tibial insert 4104, 4106 may include a slot 4160 (see FIG. 3) defined in a side wall 4162 configured to receive a portion of the rim 4132 to secure the rim 4132 to the tibial insert 4104,4106.

As shown in FIG. 4, the rotating tibial insert 4106 may be coupled to the tibial tray 4102 in a manner similar to the non-rotating tibial insert 4104. To do so, the rotating tibial insert 4106 is positioned on the upper surface 4112 of the platform 4108 such that the post 4120 is received in the aperture 4154 defined in the bottom surface 4152 of the tibial insert 4106. When so coupled, the bottom surface 4152 of the tibial insert 4106 is in contact with the upper surface 4112 of the platform 4108 of the tibial tray 4102. Because the rotating tibial insert 4106 does not include the rim 4132 and tabs 4114, the insert is free to rotate about an axis 4156 defined by the post 4120 of the tibial insert 4102. In should be appreciated that the circular shape of the post 4120 facilitates the rotation of the rotating tibial insert 4106.

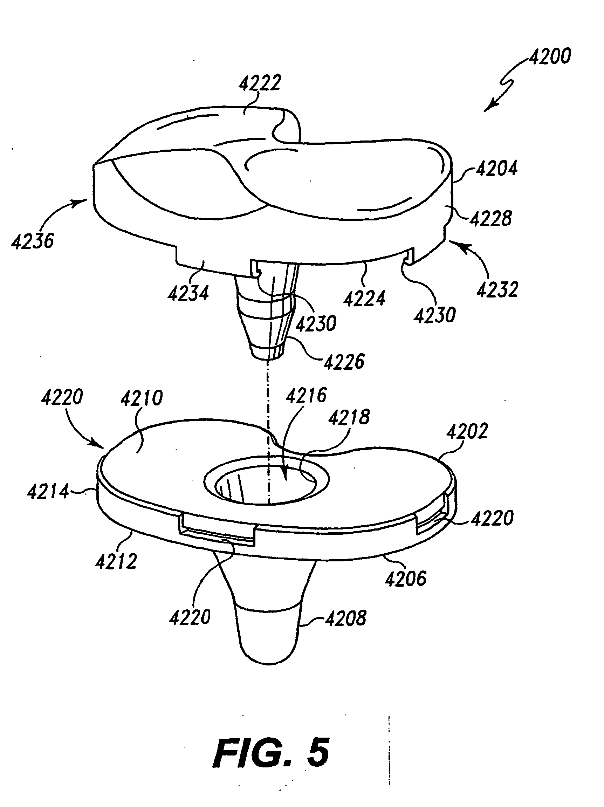

Referring now to FIG. 5, in another embodiment, a prosthetic knee system 4200 includes a tibial tray 4202, a fixed or non-rotating tibial insert 4204, and a rotating tibial insert (not shown). The tibial insert 4204 is illustratively formed from a polymer material, but may be formed from other materials, such as a ceramic material, a metallic material, a bio-engineered material, or the like, in other embodiments. Similarly, the tibial tray 4202 is illustratively formed from a metallic material, but may be formed from other materials, such as a ceramic material, a polymer material, a bio-engineered material, or the like, in other embodiments.

The tibial tray 4202 includes a platform 4206 and a stem 4208. The platform includes an upper surface 4210, a bottom surface 4212, and a side surface 4214 extending between the upper surface 4210 and the bottom surface 4212. The tibial tray 4202 also includes a cavity 4216 having an opening 4218 defined on the upper surface 4210. The stem 4208 extends downwardly from the bottom surface 4212 of the platform 4206. The platform 4206 includes a number of slots 4220 defined in the side surface 4214. Illustratively, the platform 4206 includes a slot 4220 defined in the lateral side of the side surface 4214, a slot 4220 defined in the anterior side of the side surface 4214, and a slot 4220 defined in the medial side of the side surface 4220. However, in other embodiments, the platform 4206 may include any number of slots 4220 defined in the side surface 4214.

As described above in regard to the tibial tray 4102 of FIGS. 1 to 4, the tibial tray 4202 is configured to be coupled to a surgically-prepared surface of the proximal end of a patient's tibia (not shown). When the tibial tray 4202 is so coupled, the stem 4208 is embedded in patient's tibia to thereby secure the tibial tray 4202 to the patient's bone. In some embodiments, a stem extension (not shown) may include coupled to the stem 4208 to increase the overall length of the stem 4208 and improve the stability of the tibial tray 4202 relative to the patient's bony anatomy.

The tibial insert 4204 includes an upper bearing surface 4222, a bottom surface 4224, and a stem 4226. The upper bearing surface 4222 is configured to contact a pair of natural or prosthetic femoral condyles of the patient. The stem 4226 extends downwardly from the bottom surface 4224. The tibial insert 4204 also includes a sectioned rim 4228 extending downwardly from the bottom surface 4224. The rim 4228 includes a number of tabs 4230 extending inwardly. Illustratively, the rim 4228 includes a lateral rim section 4232, an anterior rim section 4234, and a medial rim section 4336. Each section 4232, 4334, 4336 includes a separate inwardly extending tab 4230. However, in other embodiments, the rim 4228 may include more or less sections.

The tibial insert 4204 is configured to be coupled to the tibial tray 4202 in use. To do so, the tibial insert 4204 is positioned such that the stem 4206 is received in the opening 4218 defined in the upper surface 4210 of the tibial tray 4202. The tibial insert 4204 is seated on the upper surface 4210 of the platform 4206 such that the of the tabs 4230 of the rim 4228 are received in the corresponding slots 4220 defined in the side surface 4214 of the platform 4206 of the tibial tray 4202. When so coupled, the bottom surface 4224 of the tibial insert 4204 is in contact with the upper surface 4210 of the platform 4206 of the tibial tray 4202. The slots 4220 of the tibial tray 4202 and the rim 4228 and tabs 4230 of the rotating tibial insert 4204 cooperate to restrict or prevent rotation of the tibial insert 4204 relative to the tibial tray 4202, to reduce micro-motion between the tibial insert 4204 and the tibial tray 4202, and/or to prevent lift-off of the tibial insert 4204 relative to the tibial tray 4202.

A rotating tibial insert may be used with the tibial tray in place of the non-rotating tibial insert. The rotating tibial insert may be coupled to the tibial tray in a manner similar to the non-rotating tibial insert. To do so, the rotating tibial insert is positioned such that a stem of the rotating tibial insert is received in the opening defined in the upper surface of the tibial tray. Because the rotating tibial insert does not include the tabs of the non-rotating tibial insert, the insert is free to rotate about an axis defined by the post of the tibial insert.

## Claims

1. An orthopaedic prosthesis assembly (4100) comprising:
a tibial tray (4102) configured to be coupled to a surgically-prepared surface of the proximal end of a tibia, the tibial tray including a platform (4108) having a generally planar upper surface (4112), a bottom surface (4114), and a side surface (4116) extending between the upper surface and the bottom surface, the side surface having at least one slot (4118) formed in it extending at least part way around the tibial tray, and
a tibial insert (4104) coupled to the tibial tray, the tibial insert having an upper bearing surface (4126) configured to contact a pair of femoral condyles and a bottom surface (4128) in contact with the upper surface of the tibial tray, the tibial insert including a rim (4132) extending downwardly from the bottom surface of the tibial insert,
**characterised in that** the rim (4132) of the tibial insert is divided into a plurality of separate downwardly extending sections, each section having a tab (4144) extending inwardly from the rim so that, the insert can be coupled to the tray by positioning the insert on the tray and causing the bottom surface of the insert to contact the upper surface of the tray, so that the tabs on the respective sections of the rim are received in the at least one slot in the side surface of the tray and prevent the insert from lifting off the tray.

2. The orthopaedic prosthesis assembly of claim 1, in which first and second slots are formed in the side surface (4116) of the tibial tray (4102) on the anterior side of the tibial tray, and in which first and second tabs (4144) extend inwardly from the rim (4132) of the tibial insert (4104), being received in the first and second slots respectively.

3. The orthopaedic prosthesis assembly of claim 1, in which the slot (4118) in the side surface (4116) of the tibial tray (4102) defines a closed path and in which all of the tabs (4144) which are provided on the rim (4132) of the tibial insert (4104) are received in the slot.

4. The orthopaedic prosthesis assembly of claim 1, in which:
(i) the tibial tray (4102) includes a second slot and a third slot defined in the side surface (4116), the first slot being defined on the medial side of the tibial tray, the second slot being defined on the lateral side of the tibial tray, and the third slot being defined on the anterior side of the tibial tray, and
(ii) the tibial insert (4104) includes a second tab (4144) and a third tab extending inwardly from the rim, the second tab being received in the second slot and the third tab being received in the third slot.

5. The orthopaedic prosthesis assembly of claim 1, which includes a metal ring, in which:
(i) the tibial tray includes a cavity defined in the platform, the cavity being defined by a sidewall, and
(ii) the tibial insert includes a stem extending downwardly from the bottom surface and being received in the cavity of the tibial tray, the metal ring being secured to the stem, the sidewall of the cavity and the metal ring having corresponding tapers such that the sidewall of the cavity and the metal ring are in contact and form a friction lock between them.

6. The orthopaedic prosthesis assembly of claim 5, in which the taper of the sidewall and the taper of the metal ring are Morse tapers.

7. The orthopaedic prosthesis assembly of claim 1, which includes a rotating tibial insert (4104), in which the tibial insert is removably coupled to the tibial tray (4102) and the rotating tibial insert is configured to be coupled to the tibial tray when the tibial insert is removed therefrom, the rotating tibial insert being free to rotate about an axis relative to the tibial tray.

8. The orthopaedic prosthesis assembly of claim 7, in which:
(i) the tibial tray (4012) comprises a cavity (4030) defined in the platform, and
(ii) the rotating tibial insert (4016) comprises a stem (4072) extending from a bottom surface, the stem being configured to be received in the cavity of the tibial tray when the rotating tibial insert is coupled thereto.

9. The orthopaedic prosthesis assembly of claim 7 in which:
(i) the tibial tray (4102) includes a post (4120) extending upwardly from the upper surface,
(ii) the tibial insert (4104) includes an aperture (4130) defined in the bottom surface of the tibial insert, the post of the tibial tray being received in the aperture when the tibial insert is coupled to the tibial tray, and
(iii) the rotating tibial insert (4106) comprises an aperture (4154) defined in a bottom surface, the post of the tibial tray being received in the aperture when the rotating tibial insert is coupled to the tibial tray.

## Patentansprüche

1. Orthopädische Prothesenanordnung (4100), umfassend:
ein Tibiaplateau (4102), das konfiguriert ist, um mit einer chirurgisch vorbereiteten Fläche des proximalen Endes einer Tibia gekoppelt zu werden, wobei das Tibiaplateau eine Plattform (4108) mit einer allgemein planaren oberen Fläche (4112), einer unteren Fläche (4114) und einer Seitenfläche (4116), die sich zwischen der oberen Fläche und der unteren Fläche erstreckt, enthält, wobei die Seitenfläche mindestens einen Schlitz (4118) aufweist, der darin ausgebildet ist und sich zumindest teilweise um das Tibiaplateau erstreckt, und
einen Tibiaeinsatz (4104), der mit dem Tibiaplateau gekoppelt ist, wobei der Tibiaeinsatz eine obere Lagerfläche (4126), die konfiguriert ist, um ein Paar Femurkondylen zu kontaktieren, und eine untere Fläche (4128) in Kontakt mit der oberen Fläche des Tibiaplateaus aufweist, wobei der Tibiaeinsatz einen Rand (4132) enthält, der sich von der unteren Fläche des Tibiaeinsatzes nach unten erstreckt,
**dadurch gekennzeichnet, dass** der Rand (4132) des Tibiaeinsatzes in eine Vielzahl von separaten sich nach unten erstreckenden Abschnitten unterteilt ist, wobei jeder Abschnitt eine Nase (4144) aufweist, die sich von dem Rand nach innen erstreckt, sodass der Einsatz durch Positionieren des Einsatzes auf dem Plateau und bewirken, dass die untere Fläche des Einsatzes die obere Fläche des Plateaus kontaktiert, mit dem Plateau gekoppelt werden kann, sodass die Nasen an den jeweiligen Abschnitten des Randes in dem mindestens einen Schlitz in der Seitenfläche des Plateaus aufgenommen werden und verhindern, dass der Einsatz das Plateau abhebt.

2. Orthopädische Prothesenanordnung nach Anspruch 1, wobei erste und zweite Schlitze in der Seitenfläche (4116) des Tibiaplateaus (4102) auf der vorderen Seite des Tibiaplateaus ausgebildet sind und wobei sich die ersten und zweiten Nasen (4144) von dem Rand (4132) des Tibiaeinsatzes (4104) nach innen erstrecken und in dem ersten beziehungsweise zweiten Schlitz aufgenommen sind.

3. Orthopädische Prothesenanordnung nach Anspruch 1, wobei der Schlitz (4118) in der Seitenfläche (4116) des Tibiaplateaus (4102) eine geschlossene Bahn definiert und wobei alle Nasen (4144), die an dem Rand (4132) des Tibiaeinsatzes (4104) vorgesehen sind, in dem Schlitz aufgenommen sind.

4. Orthopädische Prothesenanordung nach Anspruch 1, wobei:
i) das Tibiaplateau (4102) einen zweiten Schlitz und einen dritten Schlitz enthält, die in der Seitenfläche (4116) definiert sind, wobei der erste Schlitz auf der medialen Seite des Tibiaplateaus definiert ist, der zweite Schlitz auf der lateralen Seite des Tibiaschlitzes definiert ist und der dritte Schlitz auf der vorderen Seite des Tibiaschlitzes definiert ist, und
ii) der Tibiaeinsatz (4104) eine zweite Nase (4144) und eine dritte Nase enthält, die sich von dem Rand nach innen erstrecken, wobei die zweite Nase in dem zweiten Schlitz aufgenommen ist und die dritte Nase in dem dritten Schlitz aufgenommen ist.

5. Orthopädische Prothesenanordnung nach Anspruch 1, die einen Metallring enthält, wobei:
i) das Tibiaplateau einen in der Plattform definierten Hohlraum enthält, wobei der Hohlraum von einer Seitenwand definiert wird, und
ii) der Tibiaeinsatz einen Schaft enthält, der sich von der unteren Fläche nach unten erstreckt und in dem Hohlraum des Tibiaplateaus aufgenommen ist, wobei der Metallring an dem Schaft gesichert ist, die Seitenwand des Hohlraumes und der Metallring korrespondierende Kegel aufweisen, sodass die Seitenwand des Hohlraums und der Metallring in Kontakt stehen und zwischen sich einen Reibschluss bilden.

6. Orthopädische Prothesenanordnung nach Anspruch 5, wobei der Kegel der Seitenwand und der Kegel des Metallrings Morsekegel sind.

7. Orthopädische Prothesenanordnung nach Anspruch 1, die einen Drehtibiaeinsatz (4104) enthält, wobei der Tibiaeinsatz mit dem Tibiaplateau (4102) lösbar gekoppelt ist und der Drehtibiaeinsatz konfiguriert ist, um mit dem Tibiaplateau gekoppelt zu werden, wenn der Tibiaeinsatz davon entfernt ist, wobei der Drehtibiaeinsatz um eine Achse relativ zum Tibiaplateau frei drehen kann.

8. Orthopädische Prothesenanordnung nach Anspruch 7, wobei:
i) das Tibiaplateau (4012) einen in der Plattform definierten Hohlraum (4030) aufweist, und
ii) der Tibiaeinsatz(4016) einen Schaft (4072) aufweist, der sich von einer unteren Fläche erstreckt, wobei der Schaft konfiguriert ist, um in dem Hohlraum des Tibiaplateaus aufgenommen zu werden, wenn der Drehtibiaeinsatz damit gekoppelt ist.

9. Orthopädische Prothesenanordnung nach Anspruch 7, wobei:
i) das Tibiaplateau (4102) einen Ansatz (4120) enthält, der sich von der oberen Fläche nach oben erstreckt,
ii) der Tibiaeinsatz (4104) eine in der unteren Fläche des Tibiaeinsatzes definierte Öffnung (4130) enthält, wobei der Ansatz des Tibiaplateaus in der Öffnung aufgenommen ist, wenn der Tibiaeinsatz mit dem Tibiaplateau gekoppelt ist, und
iii) der Drehtibiaeinsatz (4106) eine in einer unteren Fläche definierte Öffnung (4154) aufweist, wobei der Ansatz des Tibiaplateaus in der Öffnung aufgenommen ist, wenn der Drehtibiaeinsatz mit dem Tibiaplateau gekoppelt ist.

## Revendications

1. Ensemble de prothèse orthopédique (4100) comprenant :
un plateau tibial (4102) configuré pour être couplé à une surface préparée chirurgicalement de l'extrémité proximale d'un tibia, le plateau tibial comprenant une plateforme (4108) ayant une surface supérieure (4112) généralement plane, une surface inférieure (4114) et une surface latérale (4116) s'étendant entre la surface supérieure et la surface inférieure, la surface latérale ayant au moins une fente (4118) formée dans cette dernière s'étendant au moins partiellement autour du plateau tibial, et
un insert tibial (4104) couplé au plateau tibial, l'insert tibial ayant une surface d'appui supérieure (4126) configurée pour entrer en contact avec une paire de condyles fémoraux et une surface inférieure (4128) en contact avec la surface supérieure du plateau tibial, l'insert tibial comprenant un bord (4132) s'étendant vers le bas à partir de la surface inférieure de l'insert tibial,
**caractérisé en ce que** le bord (4132) de l'insert tibial est divisé en une pluralité de sections séparées s'étendant vers le bas, chaque section ayant une languette (4144) s'étendant vers l'intérieur à partir du bord de sorte que, l'insert peut être couplé par le plateau en positionnant l'insert sur le plateau et en amenant la surface inférieure de l'insert à entrer en contact avec la surface supérieure du plateau, de sorte que les languettes sur les sections respectives du bord sont reçues dans au moins une fente dans la surface latérale du plateau et empêchent l'insert de soulever le plateau.

2. Ensemble de prothèse orthopédique selon la revendication 1, dans laquelle des première et deuxième fentes sont formées dans la surface latérale (4116) du plateau tibial (4102) sur le côté antérieur du plateau tibial, et dans lequel des première et deuxième languettes (4144) qui s'étendent vers l'intérieur à partir du bord (4132) de l'insert tibial (4104), sont reçues dans les première et deuxième fentes respectivement.

3. Ensemble de prothèse orthopédique selon la revendication 1, dans lequel la fente (4118) dans la surface latérale (4116) du plateau tibial (4102) définit une trajectoire fermée et dans lequel la totalité des languettes (4144) qui sont prévues sur le bord (4132) de l'insert tibial (4104), sont reçues dans la fente.

4. Ensemble de prothèse orthopédique selon la revendication 1, dans lequel :
(i) le plateau tibial (4102) comprend une deuxième fente et une troisième fente définies dans la surface latérale (4116), la première fente étant définie sur le côté médian du plateau tibial, la deuxième fente étant définie sur le côté latéral du plateau tibial, et la troisième fente étant définie sur le côté antérieur du plateau tibial, et
(ii) l'insert tibial (4104) comprend une deuxième languette (4144) et une troisième languette s'étendant vers l'intérieur à partir du bord, la deuxième languette étant reçue dans la deuxième fente et la troisième languette étant reçue dans la troisième fente.

5. Ensemble de prothèse orthopédique selon la revendication 1, qui comprend un anneau métallique, dans lequel :
(i) le plateau tibial comprend une cavité définie dans la plateforme, la cavité étant définie par une paroi latérale, et
(ii) l'insert tibial comprend une tige s'étendant vers le bas à partir de la surface inférieure et étant reçue dans cavité du plateau tibial, l'anneau métallique étant fixé sur la tige, la paroi latérale de la cavité et l'anneau métallique ayant des cônes correspondants de sorte que la paroi latérale de la cavité et l'anneau métallique sont en contact et forment un verrouillage à friction entre eux.

6. Ensemble de prothèse orthopédique selon la revendication 5, dans lequel le cône de la paroi latérale et le cône de l'anneau métallique sont des cônes Morse.

7. Ensemble de prothèse orthopédique selon la revendication 1, dans lequel un insert tibial rotatif (4104), dans lequel l'insert tibial est couplé de manière amovible au plateau tibial (4102) et l'insert tibial rotatif est configuré pour être couplé au plateau tibial lorsque l'insert tibial est retiré de ce dernier, l'insert tibial rotatif étant libre de tourner autour d'un axe par rapport au plateau tibial.

8. Ensemble de prothèse orthopédique selon la revendication 7, dans lequel :
(i) le plateau tibial (4012) comprend une cavité (4030) définie dans la plateforme, et
(ii) l'insert tibial rotatif (4016) comprend une tige (4072) s'étendant à partir d'une surface inférieure, la tige étant configurée pour être reçue dans la cavité du plateau tibial lorsque l'insert tibial rotatif y est couplé.

9. Ensemble de prothèse orthopédique selon la revendication 7, dans lequel :
(i) le plateau tibial (4102) comprend un montant (4120) s'étendant vers le haut à partir de la surface supérieure,
(ii) l'insert tibial (4104) comprend une ouverture (4130) définie dans la surface inférieure de l'insert tibial, le montant du plateau tibial étant reçu dans l'ouverture lorsque l'insert tibial est couplé au plateau tibial, et
(iii) l'insert tibial rotatif (4106) comprend une ouverture (4154) définie dans une surface inférieure, le montant du plateau tibial étant reçu dans l'ouverture lorsque l'insert de plateau tibial rotatif est couplé au plateau tibial.
